# EUROPEAN PATENT APPLICATION

(11) **EP 4 111 944 A1**
(43) Date of publication of application: **04.01.2023**
(21) Application number: 21182690.4
(22) Date of filing: 30.06.2021
(51) Int. Cl.: A61B 5/00, G01S 5/02, H04W 4/029, H04W 4/33

(54) **ESTIMATING AN ACTUAL LIGHT PROFILE FOR AN INDIVIDUAL**

(71) Applicant: BrainLit AB, 223 63 Lund (SE)
(72) Inventor: Lindoff, Bengt, Bjärred (SE); Wingren, Tord, Lomma (SE); Silfverström, Niklas, Helsingborg (SE); Andersson, Jacob, Helsingborg (SE)
(74) Representative: AWA Sweden AB

(57) **Abstract**

The present inventive concept relates to a portable electronic device (10), a server (20), and a method (30) for estimating an actual light profile for an individual. The method (30) comprising: determining (S300), by a portable electronic device (10) associated with the individual, whether the individual is presently indoors or outdoors; and upon the individual is determined (S300A) to be indoors: determining (S302) a specific building in which the individual is presently present, and accumulating (S306) predetermined indoor light exposure data associated with the specific building to the actual light profile of the individual, thereby estimating the actual light profile for the individual.

## Description

### Technical field

The present inventive concept relates to a method for estimating an actual light profile for an individual.

### Background of the invention

Chronobiology, which is a field of biology that studies timing (e.g., periodic) processes and phenomena in living organisms, is becoming increasingly more important for understanding human physiology. For example, the circadian rhythm is an internal process of living organisms (e.g. humans) that regulates the sleep-wake cycle. The circadian rhythm typically repeats over a time period which, for most people, is slightly longer than 24 hours. It is also known that this cycle can be different between different individuals. For example, some persons are known to be "morning" persons, while other are "evening" persons. Hence, the behavior at a certain time of day for different individual typically varies. This is usually referred to as a person's chronotype.

An important part of chronobiology is the study of the impact of light on living organisms. It has, for example, been found that exposing a person to bright light in the evening can postpone the time that person falls asleep. Recent studies have further found that light can affect a person's alertness and performance. Nowadays, light treatment is starting to be utilized by persons wanting to align their peak performance with an important event, for example an athlete wanting to perform the best in a competition. However, it has been found that the effect of light at a certain time of the day is not only instant, it also depends on the individual's prior exposure to light during the day. For instance, in case an individual has been exposed to bright light in the morning (e.g., light similar to that of a bright summer's morning), the effect of bright light during the afternoon is typically less than if the same person had spent the morning being exposed to light of lower illuminance (e.g. artificial light inside an office).

It is therefore important to know the light history of an individual. This is typically done by recording the light exposure of the individual using wearable light sensors. However, such solutions are prone to errors, since the individual may forget to put on the light sensor, or the sensor is worn incorrectly (e.g. being blocked by clothing). This results in an inaccurate light history, and, in turn, optimal light setting for the individual cannot be determined properly. Therefore, there exists a need for better knowledge regarding an individual's light history.

### Summary of the invention

It is an object to, at least partly, mitigate, alleviate, or eliminate one or more of the above-identified deficiencies in the art and disadvantages singly or in any combination and solve at least the above-mentioned problem.

According to a first aspect a method for estimating an actual light profile for an individual is provided. The method comprising: determining, by a portable electronic device associated with the individual, whether the individual is presently indoors or outdoors; and upon the individual is determined to be indoors: determining a specific building in which the individual is presently present, and accumulating predetermined indoor light exposure data associated with the specific building to the actual light profile of the individual, thereby estimating the actual light profile for the individual.

Within the context of this disclosure, the wording "actual light profile" should be construed as spectrally resolved information/data associated with an accumulated amount of light that the user has been exposed to during a certain time range (e.g. during the day, week, and/or month). Since the information/data is spectrally resolved, the actual light profile may comprise information of the amount of light at which wavelength/color the user has been exposed to. Put differently, the actual light profile may be the light history of the user.

Within the context of this disclosure, the wording "predetermined indoor light exposure data" should be construed as data associated to a lighting environment in the associated specific building. Since it is predetermined, the data is determined at a prior occasion. The predetermined indoor light exposure data may, e.g., be determined (e.g. by measuring using a light sensor) when a lighting system is commissioned in the specific building or subsequent to the commissioning. The predetermined indoor light exposure data may be an estimation of the light environment inside the specific building. The predetermined indoor light exposure may be associated with the type of location (e.g. a residential building, an office building, etc.). The lighting environment inside the specific building may be estimated or simulated using information associated with positions and light emitted by light sources arranged inside the specific building. Further, the predetermined indoor light exposure data may be associated with a default indoor lighting environment. The default lighting environment may be a typical indoor lighting environment. The default lighting environment may, e.g., be an average of lighting environments of a plurality of different building. The predetermined indoor light exposure data may comprise information on an intensity and/or a spectral distribution of light emitted by lights in the specific building. The lighting environment inside the specific building may vary depending on time of day and/or between different rooms. Hence, the predetermined indoor light exposure data may be an average of the lighting environment for different times of day and/or different rooms of the specific building.

By means of the present method, the actual light profile (i.e. light history) of the user can be estimated without the individual having to carry a light sensor. In particular, if the individual is already carrying the portable electronic device, which may be the case for personal electronic devices (e.g. smartphones, smartwatches, etc.), the actual light profile is estimated without the need for additional devices and/or sensors. A further advantage of the present method is that the light emitted by the lighting system of the specific building need not be monitored, e.g. by being connected to a central server which monitors the light output of the light sources of the lighting system. Hence, the present method allows for the estimation of an actual light profile for an individual presently present in a building having a conventional lighting system (i.e. with non-connected light sources).

Determining whether the individual is presently indoors or outdoors may comprise sensing a global position variation of a global position determined by a global position sensor of the portable electronic device; and wherein the individual may be determined to be presently indoors upon the global position variation being above a predetermined threshold and to be presently outdoors upon the global position variation being at or below the predetermined threshold.

Within the context of this disclosure, the wording "global position variation" should be construed as a variation over a predetermined time period of global positions determined by the global position sensor of the portable electronic device. The global position of the portable electronic device may be a longitude and/or a latitude of the portable electronic device. In that sense, it should not be construed as a true global position of the portable device. For example, in case the portable electronic device is stationary, the global position variation may, e.g., depend on the type of the location in which the portable electronic device is presently present. In particular, the global position variation may be high in case the portable electronic device is indoors than in case it is outdoors. For example, in case the global position sensor communicates with satellites and/or mobile phone masts to determine the global position of the portable electronic device, a signal strength of the communication may be degraded if the communication is blocked (e.g. by being inside a building). The variation may be represented by a variance of different global positions determined over a predetermined time period. It is to be understood that other representations of variation are known to the skilled person, such as standard deviation, variance, and other variation metrics etc. For example, the global position variation may be based on a distance between percentiles of the different global positions determined over the predetermined time period.

Since the determination of the global position of the portable electronic device may be less precise in case the portable electronic device is inside a building (i.e. indoors) than in case the portable electronic device is outdoors, the variation of the global position may be a robust measure whether the portable electronic device (and hence the individual) is indoors or outdoors. This is in particular advantageous in case the portable electronic device is a personal electronic device that individual is already carrying.

The predetermined indoor light exposure data may be time resolved, the method further comprising: determining a present time; and wherein the actual light profile may be estimated by accumulating predetermined indoor light exposure data associated with the specific building and the present time.

An associated advantage is that the estimated actual light profile may be more in line with the actual light exposure of the individual.

The method may further comprise: retrieving the predetermined indoor light exposure data from a database comprising entries correlating buildings and predetermined indoor light exposure data.

An associated advantage is that the actual light profile may be estimated even if the individual visits more than one building (i.e. for all buildings in the database). Hence, a better estimation of the actual light profile may be allowed.

The method may further comprise: upon the individual is determined to be outdoors: determining, by a global position sensor of the portable electronic device, a global position at which the individual is presently present, determining a present time, and accumulating outdoor light exposure data associated with the global position and the present time to the actual light profile of the individual.

Within the context of this disclosure, the wording "outdoor light exposure data" should be construed as data associated to an outdoor lighting environment. The outdoor light exposure data may be determined using weather data associated with the global position at which the individual is presently present. The outdoor lighting environment may be estimated or simulated using information associated with the global position of the portable electronic device and/or the present time. Further, the outdoor light exposure data may be associated with a default outdoor lighting environment. The default outdoor environment may be a typical outdoor lighting environment. The default outdoor lighting environment may, e.g., be an average of lighting environments of a plurality of different global positions. The outdoor light exposure data may comprise information on an intensity and/or a spectral distribution of light at the global position of the portable device. The outdoor lighting environment may vary depending on time of day.

An associated advantage is that the actual light profile for the individual may account for periods of time that the individual is outdoors. Hence, a better estimation of the actual light profile may be allowed.

The method may further comprise: retrieving the outdoor light exposure data from a server on which outdoor light exposure data is stored and/or from a database comprising entries correlating global positions, times, and outdoor light exposure data.

According to a second aspect, a portable electronic device is provided. The portable electronic device comprising: circuitry configured to: determine whether the portable electronic device is presently indoors or outdoors; and upon the portable device is determined to be indoors: determine a specific building in which the portable electronic device is presently present, and accumulate predetermined indoor light exposure data associated with the specific building to an actual light profile for an individual associated with the portable electronic device.

The above-mentioned features of the first aspect, when applicable, apply to this second aspect as well. In order to avoid undue repetition, reference is made to the above.

The portable electronic device may further comprise: a global position sensor configured to determine a global position of the portable electronic device; and wherein the circuitry may be further configured to: sense a global position variation of the global position determined by the global position sensor, and determine that the portable electronic device is presently indoors upon the global position variation being above a predetermined threshold and presently outdoors upon the global position variation being at or below the predetermined threshold.

The predetermined indoor light exposure data may be time resolved, and the portable device may further comprise: a clock configured to determine a present time; and wherein the circuitry may be further configured to: accumulate predetermined indoor light exposure data associated with the specific building and the present time.

The circuitry may be further configured to: retrieve the predetermined indoor light exposure data from a database comprising entries correlating building and predetermined indoor light exposure data.

The portable electronic device may further comprise: a global position sensor configured to determine a global position of the portable electronic device; a clock configured to determine a present time; and wherein the circuitry may be further configured to: upon the portable electronic device is determined to be outdoors: accumulate outdoor light exposure data associated with the global position at which the portable electronic device is presently present and the present time to the actual light profile.

According to a third aspect a server is provided. The server comprising: circuitry configured to: receive a signal associated with a location of a portable electronic device, wherein the portable electronic device is associated with an individual; determine a variation of the received signal; determine the portable electronic device to be presently indoors or outdoors by comparing the signal variation with a predetermined value; and upon the portable electronic device is determined to be indoors: determine a specific building in which the portable device is presently present, and accumulate predetermined indoor light exposure data associated with the specific building to an actual light profile for the individual associated with the portable electronic device.

The above-mentioned features of the first and second aspects, when applicable, apply to this third aspect as well. In order to avoid undue repetition, reference is made to the above.

The received signal may be associated with a present global position of the portable electronic device, and wherein the circuitry may be further configured to: determine the portable electronic device to be presently indoors upon the signal variation being above a predetermined threshold and to be presently outdoors upon the signal variation being at or below the predetermined threshold.

The predetermined indoor light exposure data may be time resolved, and the server may further comprise: a clock configured to determine a present time; and wherein the circuitry may be further configured to: accumulate predetermined indoor light exposure data associated with the specific building and the present time.

The circuitry may be further configured to: retrieve the predetermined indoor light exposure data from a database comprising entries correlating buildings and predetermined indoor light exposure data.

The server may further comprise: a clock configured to determine a present time; and wherein the circuitry may be further configured to: upon the portable electronic device is determined to be outdoors: accumulate outdoor light exposure data associated with the global position and the present time to the actual light profile for the individual.

A further scope of applicability of the present disclosure will become apparent from the detailed description given below. However, it should be understood that the detailed description and specific examples, while indicating preferred variants of the present inventive concept, are given by way of illustration only, since various changes and modifications within the scope of the inventive concept will become apparent to those skilled in the art from this detailed description.

Hence, it is to be understood that this inventive concept is not limited to the particular steps of the methods described or component parts of the systems described as such method and system may vary. It is also to be understood that the terminology used herein is for purpose of describing particular embodiments only and is not intended to be limiting. It must be noted that, as used in the specification and the appended claim, the articles "a", "an", "the", and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings do not exclude other elements or steps.

### Brief description of the drawings

The above and other aspects of the present inventive concept will now be described in more detail, with reference to appended drawings showing variants of the inventive concept. The figures should not be considered limiting the inventive concept to the specific variant; instead they are used for explaining and understanding the inventive concept. As illustrated in the figures, the sizes of layers and regions are exaggerated for illustrative purposes and, thus, are provided to illustrate the general structures of variants of the present inventive concept. Like reference numerals refer to like elements throughout.
Figure 1 illustrates a portable electronic device.
Figure 2 illustrates a server and a portable electronic device.
Figure 3 is a block scheme of a method for estimating an actual light profile for an individual.

### Detailed description

The present inventive concept will now be described more fully hereinafter with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

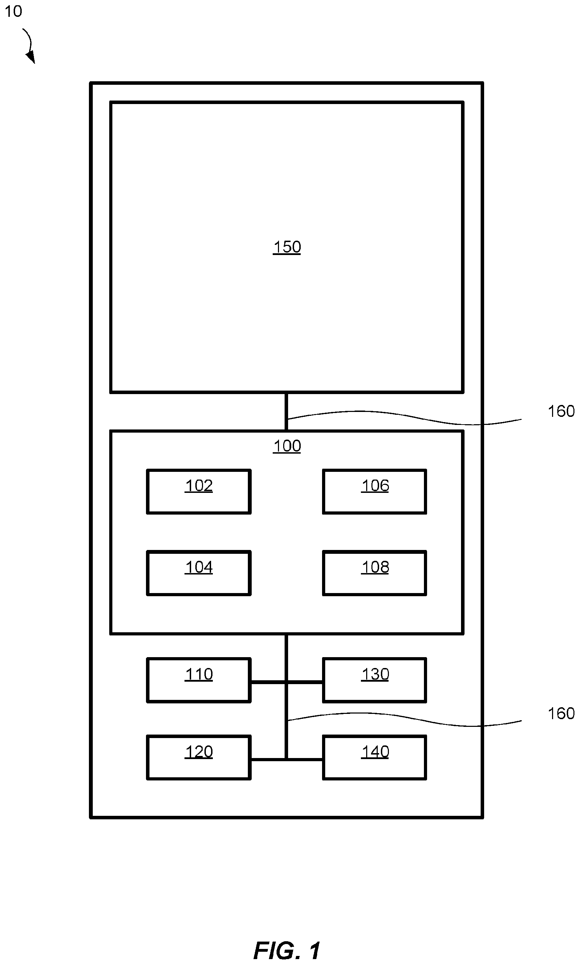

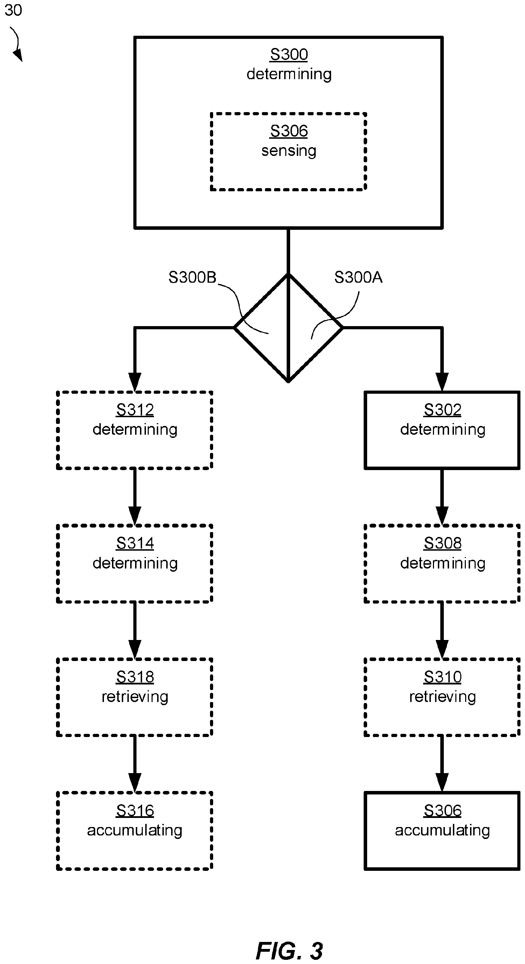

A portable electronic device 10 and a method 30 for estimating an actual light profile of an individual will now be described with reference to Fig. 1 and Fig. 3.

Figure 1 illustrates a portable electronic device 10. As illustrated in the example of Fig. 1, the portable electronic device 10 may be a smartphone. However, it is to be understood that the portable electronic device 10 may be a different device. For instance, it may be a smartwatch, a smartring, a laptop, etc. As illustrated in Fig. 1, the portable electronic device 10 comprises circuitry 100. The portable electronic device 10 may further comprise one or more of a global position sensor 110, a clock 120, an accelerometer 130, and a gyro 140 as shown in the example of Fig. 1. As illustrated in Fig. 1, the portable electronic device 10 may further comprise a screen 150, such as a touch screen. The screen 150 may be configured as an input interface allowing a user to interact with different functions of the portable electronic device 10. The screen 150 may further be configured to display information to the user of the portable electronic device 10. One or more of the circuitry 100, the global position sensor 110, the clock 120, the accelerometer 130, the gyro, and the screen 150 may communicate via a data bus 160. The global position sensor 110 may be configured to determine a global position of the portable electronic device 10. The global position sensor 110 may be configured to use a Global Navigation Satellite System (GNSS). Examples of GNSS comprise Global Positioning System (GPS), Galileo positioning system, Global Navigation Satellite System (GLONASS), and BeiDou, to name a few. The clock 120 may be configured to determine S308 a present time.

The circuitry 100 may comprise a non-transitory computer-readable storage medium 102. The circuitry 100 may comprise a processor 104. The non-transitory computer-readable storage medium 102 may store program code portions that, when executed by the processor 104, performs one or more functions of the circuitry 100. Put differently, the circuitry 100 may be configured to perform a function by having program code portions stored on the non-transitory computer-readable storage medium 102 that, when executed by the processor 104, causes the circuitry 100 to perform the function.

The circuitry 100 may further comprise a transmitter 106 and/or a receiver 108. The transmitter 106 and/or receiver 108 may be configured for wireless communication. Hence, the transmitter 106 and/or receiver 108 may allow the portable electronic device 10 to communicate with other electronic devices. The transmitter 106 and/or receiver 108 may be configured for short-range communication (e.g. Bluetooth, NFC, Wi-Fi, etc.) and/or long-range communication (3G, 4G, 5G, LTE, satellite, etc.).

The circuitry 100 is configured to determine S300 whether the portable electronic device 10 is presently indoors or outdoors.

The circuitry 100 may be configured to determine S300 whether the portable electronic device 10 is presently indoors or outdoors in a few different ways. For example, the circuitry 100 may be configured to sense a movement of the portable electronic device 10. The movement may be determined by one or more of the global position sensor 110, the accelerometer 130, and the gyro 140 of the portable electronic device 10. For example, in case the portable electronic device 10 is outdoors, the movement of the portable electronic device 10 may be higher than in case the portable electronic device 10 is indoors. Further the circuitry 100 may be configured to sense a variation of the movement of the portable electronic device 10. The movement variation of the portable electronic device 10 may be analyzed by the circuitry 100 to determine whether the portable electronic device 10 is presently indoors or outdoors. For example, the movement variation may be at or above a predetermined movement variation threshold in case the portable electronic device 10 is outdoors and below the predetermined movement threshold in case the portable electronic device 10 is indoors. Another example of how the circuitry 100 may determine S300 whether the portable device 10 is presently indoors or outdoors will now be described. In this example, the circuitry 100 may be further configured to sense S306 a global position variation of the global position determined by the global position sensor 110. The global position variation of the portable electronic device 10 may be analyzed by the circuitry 100 to determine whether the portable electronic device 10 is presently indoors or outdoors. In particular, an accuracy associated with the global position determined by the global position sensor 110 of the portable electronic device 10 may vary depending on whether the portable electronic device 10 is presently indoors or outdoors. Typically, in case the portable electronic device 10 is outdoors the global position sensor 110 may determine the global position of the portable electronic device 10 with higher accuracy than in case the portable electronic device 10 is indoors. This since the signals used for determining the position by the global position sensor may be of weaker signal strength indoors compared to outdoors, which may give rise to more signal noise and thereby an increased global position variation. Hence, a high global position variation may be associated with a low accuracy of the determined global position, which, in turn, indicates that the portable electronic device 10 may be indoors. Likewise, a low global position variation may be associated with a high accuracy of the determined global position, which, in turn, indicates that the portable electronic device 10 may be outdoors. Hence, the circuitry 100 may be further configured to determine S300 that the portable electronic device 10 is presently indoors upon the global position variation being above a predetermined threshold and presently outdoors upon the global position variation being at or below the predetermined threshold.

The circuitry 100 is further configured to, upon the portable device 10 is determined S300A to be indoors, determine S302 a specific building in which the portable electronic device 10 is presently present. The circuitry 100 may use the global position determined by the global position sensor 110 to determine which specific building the portable electronic device 10 is presently present. As discussed above, the accuracy of the global position may be relatively high when the portable electronic device 10 is indoors (compared to outdoors), and the circuity may therefore use an approximate global position of the portable electronic device 10 to determine which specific building that the portable electronic device 10 is presently present in. The approximate global position may be a mean or an average of different global positions determined over a predetermined time period. The skilled person knows that there are different ways in which the mean or the average may be determined, such as weighted means, arithmetic means, geometric means, harmonic means, etc. Further, the above examples may use a median instead of a mean. A building database comprising entries correlating specific buildings with global positions and/or approximate global positions may be accessible to the portable electronic device 10. The building database may be accessible to the circuitry 100 by being stored on the non-transitory computer-readable storage medium 102 of the circuitry 100 and/or by the circuitry 100 communicating, using the transmitter 106/receiver 108, with, e.g., a server on which the building database is stored. Thus, the circuitry 100 may determine by comparing the global position (or the approximate global position) with the entries of the building database to determine the specific building. Further, the circuitry 100 may determine the specific building based on short-range communication. For example, the specific building may be associated with one or more Wi-Fi networks, and the circuitry 100 may be configured to determine the specific building, and/or a floor in the specific building, in which it is presently present by identifying and/or connecting to the one or more Wi-Fi networks. Hence, the building database may additionally, or alternatively, comprise entries correlating buildings and/or floors in buildings with Wi-Fi networks. A skilled person realizes that other short-range communication technologies may be used instead or in addition to Wi-Fi as described above. For example, a specific building may comprise a Bluetooth beacon configured to broadcast its presence, which the circuitry 100 may detect and thereby determine the specific building in which the portable electronic device 10 is presently present. In case the circuitry 100 is unable to determine which specific building that the portable electronic device 10 is presently present in, a default building may be used.

The circuitry 100 is further configured to accumulate S306 predetermined indoor light exposure data associated with the specific building to an actual light profile for an individual associated with the portable electronic device 10. The individual may be a human. The predetermined indoor light exposure data may be data associated with an intensity and/or a spectral distribution of an indoor lighting environment of the specific building. The indoor lighting environment may be associated with light emitted by one or more light sources arranged in the specific building. The indoor lighting environment may be further associated with all light inside the specific building. For example, the predetermined indoor light exposure data may further account for outdoor light which enters the specific building through, e.g., windows. In case no predetermined indoor light exposure data is associated with the specific building in which the portable device is presently present and/or in case the circuitry 100 is unable to determine which specific building that the portable electronic device 10 is presently present, a default predetermined indoor light exposure data may be used. The default predetermined indoor light exposure data may be associated with an average and/or a typical indoor lighting environment. The default indoor light exposure data may be different for different types of buildings (e.g. residential buildings, hospitals, airports, etc.). Hence, the building database may comprise information associated with the type of the specific building without necessarily having a predetermined indoor light exposure stored for that specific building.

The predetermined indoor light exposure data may be time resolved. Typically, the lighting environments in buildings are different depending on a time of day. For example, an amount of natural light (i.e. light from outside entering the building through, e.g., a window) varies depending on the time of day. Further, an intensity and/or a color (i.e. spectral distribution) of light emitted by light sources arranged in the specific building may change depending on the time of day. Therefore, the circuitry 100 may be further configured to accumulate predetermined indoor light exposure data associated with the specific building and the present time determined by the clock 120. The predetermined indoor light exposure data may, e.g., be stored in a database. This database and the building database discussed above may be the same database. The database may comprise entries correlating buildings and predetermined indoor light exposure data. The database may be stored on non-transitory computer-readable storage medium 102 of the circuitry 100 of the portable electronic device 10. The database may be stored on a server (e.g. a cloud server) and the circuitry 100 may communicate (via the transmitter 106 and/or receiver 108) with the server to retrieve the database and/or the predetermined indoor light exposure data. Hence, the circuitry 100 may be further configured to retrieve S31 0 the predetermined indoor light exposure data from a database comprising entries correlating buildings and predetermined indoor light exposure data.

The circuitry 100 may be further configured to, upon the portable electronic device 10 is determined S300B to be outdoors, accumulate S316 outdoor light exposure data associated with the global position at which the portable electronic device 10 is presently present and the present time to the actual light profile. The circuitry 100 may be configured to retrieve S318 (e.g. using the receiver 108 and/or transmitter 106) the outdoor light exposure data from a server on which outdoor light exposure data is stored and/or from a database comprising entries correlating global positions, times, and outdoor light exposure data. The server may, e.g., be a cloud server, or a local server. The outdoor light exposure data may be time resolved. The outdoor light exposure data may be determined or estimated from weather data associated with the global position of the portable electronic device 10. The weather data may be retrieved from an online weather service provider.

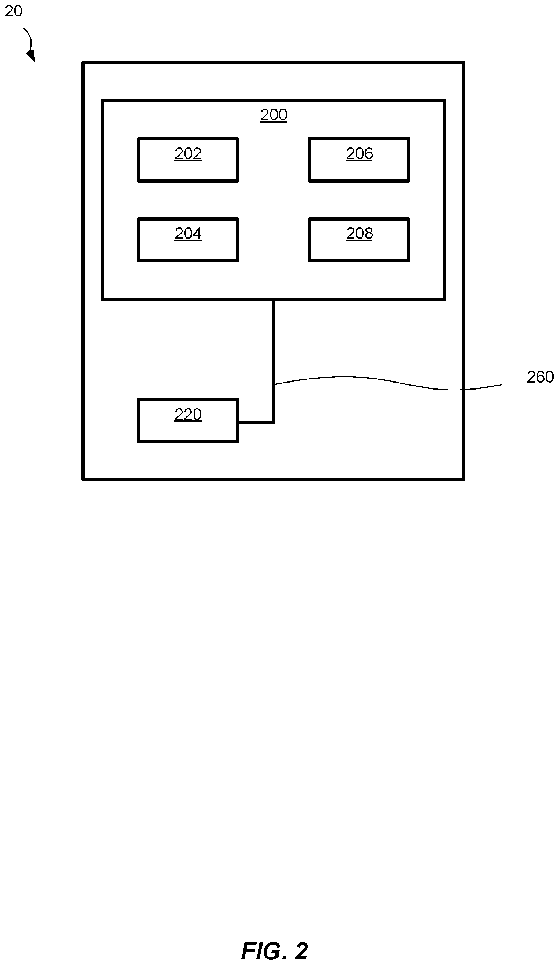

In connection to the description of Fig. 1 and Fig. 3, the circuitry 100 of the portable electronic device 10 has been described as performing the estimation of the actual light profile. However, it is to be understood that the portable electronic device 10 may communicate with a server which is configured to estimate the actual light profile for the individual. Such server is illustrated in Fig. 2 and will now be briefly described. Several of the features of the portable electronic device 10 described in connection with Fig. 1 and Fig. 3 are applicable to the server 20 illustrated in Fig. 2. In particular, the circuitry 200 of the server 20 illustrated in Fig. 2 may comprise several features of the circuitry 100 of the portable electronic device 10 illustrated in Fig. 1. To avoid undue repetition, reference is therefore made to the above.

As is shown in the example of Fig. 2, the server 20 comprises circuitry 200. The server 20 may further comprise a clock 220 configured to determine a present time. The circuitry 200 and the clock 220 may be configured to communicate via data bus 260. The circuitry 200 of the server 20 may further comprise a transmitter 206 and/or a receiver 208 allowing the circuitry 200 to communicate with other electronic devices (e.g. the portable electronic device 20). The transmitter 206 and/or receiver 208 may be configured to short-range communication and/or long-range communication.

The circuitry 200 of the server 20 is configured to receive a signal associated with a location of a portable electronic device, and to determine a variation of the received signal. The portable electronic device of the example of Fig. 2 is associated with an individual and may be similar to the portable electronic device 10 of Fig. 1. However, in the example of Fig. 2, as will be described below, the server 20 estimates the actual light profile for the individual associated with the portable electronic device, and the portable electronic device may therefore only need to provide a signal associated with its location to the server 20. Put differently, the portable electronic device of the example in Fig. 2 may not need all features of the portable electronic device 10 of Fig. 1. The circuitry 200 of the server 20 is further configured to determine the portable electronic device to be presently indoors or outdoors by comparing the signal variation with a predetermined value.

The signal may, similar to the discussion in connection to Fig. 1, be associated with a movement of the portable electronic device. Hence, the portable electronic device of the example of Fig. 2 may comprise an accelerometer and/or a gyro. The discussion in connection with Fig. 1 of how the movement of the portable electronic device 10 may be associated whether the portable electronic device 10 is indoors or outdoors may apply to this example as well. As a different example, the received signal may be associated with a present global position of the portable electronic device, and the circuitry 200 of the server 20 may be further configured to determine the portable electronic device to be presently indoors upon the signal variation being above a predetermined threshold and to be presently outdoors upon the signal variation being at or below the predetermined threshold. Again, the corresponding discussion in connection to Fig. 1 may apply to this example as well.

The circuitry 200 of the server 20 is further configured to, upon the portable electronic device is determined to be indoors, determine a specific building in which the portable device is presently present, and accumulate predetermined indoor light exposure data associated with the specific building to an actual light profile for the individual associated with the portable electronic device. The predetermined indoor light exposure data may be time resolved, and the circuitry 200 of the server 20 may be further configured to accumulate predetermined indoor light exposure data associated with the specific building and the present time. The circuitry 200 of the server 20 may be further configured to retrieve the predetermined indoor light exposure data from a database comprising entries correlating buildings and predetermined indoor light exposure data. Similar to the example of Fig. 1, the database may be stored on the non-transitory computer-readable storage medium 202 of the circuitry 200 of the server 20. The database may be stored on a different server (e.g. a cloud server) and the server 20 may retrieve information associated with the database by communicating with the different server using the transmitter 206 and/or receiver 208. The circuitry 200 of the server 20 may further comprise a processor 204. Further, the circuitry 200 of the server 20 may be configured to perform a task by the non-transitory computer-readable storage medium 202 comprising program code portions that, when executed by the processor 204, causes the circuitry to perform the task.

The circuitry 200 of the server 20 may be further configured to, upon the portable electronic device is determined to be outdoors, accumulate outdoor light exposure data associated with the global position and the present time to the actual light profile for the individual.

Hence, as described in connection with Fig. 2, the server 20 may estimate the actual light profile for the individual, and the portable electronic device associated with the individual may provide a signal associated with its location (e.g., a global position variation).

The person skilled in the art realizes that the present inventive concept by no means is limited to the preferred variants described above. On the contrary, many modifications and variations are possible within the scope of the appended claims.

For example, it is described above that either the portable electronic device or the server performs certain steps in the estimation of the actual light profile for the individual. However, it is to be understood that the steps of the method may be distributed between the portable electronic device and the server. For example, the portable electronic device may be configured to determine whether the portable electronic device is presently indoor or outdoors, and the server may be configured to determine which specific building the portable electronic device is in and to estimate the actual light profile.

Additionally, variations to the disclosed variants can be understood and effected by the skilled person in practicing the claimed invention, from a study of the drawings, the disclosure, and the appended claims.

## Claims

1. A method (30) for estimating an actual light profile for an individual, the method (30) comprising:
determining (S300), by a portable electronic device (10) associated with the individual, whether the individual is presently indoors or outdoors; and
upon the individual is determined (S300A) to be indoors:
determining (S302) a specific building in which the individual is presently present, and
accumulating (S306) predetermined indoor light exposure data associated with the specific building to the actual light profile of the individual, thereby estimating the actual light profile for the individual.

2. The method (30) according to claim 1, wherein determining (S300) whether the individual is presently indoors or outdoors comprises sensing (S306) a global position variation of a global position determined by a global position sensor (110) of the portable electronic device (10); and
wherein the individual is determined (S300) to be presently indoors upon the global position variation being above a predetermined threshold and to be presently outdoors upon the global position variation being at or below the predetermined threshold.

3. The method (30) according to any one of claims 1 or 2, wherein the predetermined indoor light exposure data is time resolved, the method (30) further comprising:
determining (S308) a present time; and
wherein the actual light profile is estimated by accumulating (S306) predetermined indoor light exposure data associated with the specific building and the present time.

4. The method (30) according to any one of claims 1-3, further comprising:
retrieving (S310) the predetermined indoor light exposure data from a database comprising entries correlating buildings and predetermined indoor light exposure data.

5. The method (30) according to any one of claims 1-4, further comprising:
upon the individual is determined (S300B) to be outdoors:
determining (S312), by a global position sensor (110) of the portable electronic device (10), a global position at which the individual is presently present,
determining (S314) a present time, and
accumulating (S316) outdoor light exposure data associated with the global position and the present time to the actual light profile of the individual.

6. A portable electronic (10) device comprising:
circuitry (100) configured to:
determine whether the portable electronic device (10) is presently indoors or outdoors; and
upon the portable device (10) is determined to be indoors:
determine a specific building in which the portable electronic device (10) is presently present, and
accumulate predetermined indoor light exposure data associated with the specific building to an actual light profile for an individual associated with the portable electronic device (10).

7. The portable electronic device (10) according to claim 6, further comprising:
a global position sensor (110) configured to determine a global position of the portable electronic device (10); and
wherein the circuitry (100) is further configured to:
sense a global position variation of the global position determined by the global position sensor (110), and
determine that the portable electronic device (10) is presently indoors upon the global position variation being above a predetermined threshold and presently outdoors upon the global position variation being at or below the predetermined threshold.

8. The portable electronic device (10) according to claim 6 or 7, wherein the predetermined indoor light exposure data is time resolved, the portable device (10) further comprising:
a clock (120) configured to determine a present time; and
wherein the circuitry (100) is further configured to:
accumulate predetermined indoor light exposure data associated with the specific building and the present time.

9. The portable electronic device (10) according to any one of claims 6-8, wherein the circuitry (100) is further configured to:
retrieve the predetermined indoor light exposure data from a database comprising entries correlating building and predetermined indoor light exposure data.

10. The portable electronic device (10) according to any one of claims 6-9, further comprising:
a global position sensor (110) configured to determine a global position of the portable electronic device;
a clock (120) configured to determine a present time; and
wherein the circuitry (100) is further configured to:
upon the portable electronic device (10) is determined to be outdoors:
accumulate outdoor light exposure data associated with the global position at which the portable electronic device (10) is presently present and the present time to the actual light profile.

11. A server (20) comprising:
circuitry (200) configured to:
receive a signal associated with a location of a portable electronic device, wherein the portable electronic device is associated with an individual;
determine a variation of the received signal;
determine the portable electronic device to be presently indoors or outdoors by comparing the signal variation with a predetermined value; and
upon the portable electronic device is determined to be indoors:
determine a specific building in which the portable device (20) is presently present, and
accumulate predetermined indoor light exposure data associated with the specific building to an actual light profile for the individual associated with the portable electronic device.

12. The server (20) according to claim 11, wherein the received signal is associated with a present global position of the portable electronic device, and wherein the circuitry (200) is further configured to:
determine the portable electronic device to be presently indoors upon the signal variation being above a predetermined threshold and to be presently outdoors upon the signal variation being at or below the predetermined threshold.

13. The server (20) according to claim 11 or 12, wherein the predetermined indoor light exposure data is time resolved, the server (20) further comprising:
a clock (220) configured to determine a present time; and
wherein the circuitry (200) is further configured to:
accumulate predetermined indoor light exposure data associated with the specific building and the present time.

14. The server (20) according to any one of claims 11-13, wherein the circuitry (200) is further configured to:
retrieve the predetermined indoor light exposure data from a database comprising entries correlating buildings and predetermined indoor light exposure data.

15. The server (20) according to any one of claims 12-14, wherein the received signal is associated with a present global position of the portable electronic device, the server (20) further comprising:
a clock (220) configured to determine a present time; and
wherein the circuitry (200) is further configured to:
upon the portable electronic device is determined to be outdoors:
accumulate outdoor light exposure data associated with the global position and the present time to the actual light profile for the individual.
